# EUROPEAN PATENT APPLICATION

(11) **EP 4 212 543 A1**
(43) Date of publication of application: **19.07.2023**
(21) Application number: 21864486.2
(22) Date of filing: 29.01.2021
(51) Int. Cl.: C07K 14/005, A61K 39/215, A61P 31/14

(54) **FUSION PROTEIN COMPRISING CORONAVIRUS-DERIVED RECEPTOR BINDING DOMAIN AND NUCLEOCAPSID PROTEIN, AND USE THEREOF**

(30) Priority: 07.09.2020 KR 20200113989
(71) Applicant: GI Cell, Inc., Seongnam-si, Gyeonggi-do 13201 (KR)
(72) Inventor: JANG, Myoung Ho, Seoul 05849 (KR); PARK, Jae Chan, Seoul 05853 (KR); CHOI, Young Joo, Hwaseong-si, Gyeonggi-do 18479 (KR); PARK, Young Hyun, Seoul 08365 (KR); KIM, Gil-Jung, Seongnam-si, Gyeonggi-do 13506 (KR); JEONG, Seung Mi, Seongnam-si, Gyeonggi-do 13362 (KR); LEE, Su Bin, Daejeon 34232 (KR)
(74) Representative: Mewburn Ellis LLP
(86) International application number: PCT/KR2021/001245
(87) International publication number: WO 2022/050520

(57) **Abstract**

The present invention relates to a fusion protein comprising a SARS-CoV-2-derived receptor-binding domain and a nucleocapsid protein, and the use thereof. The fusion protein comprising a coronavirus-derived receptor-binding domain and a nucleocapsid protein is highly applicable to a multivalent vaccine composition having greatly improved in-vivo half-life and remarkably superior efficacy compared to an immunogenic composition comprising only a receptor-binding domain. In particular, the fusion protein can greatly improve the titer of the coronavirus-specific antibody formation and T-cell immune response, and is thus useful for the prevention and treatment of coronaviruses comprising SARS-CoV-2.

## Description

### BACKGROUND OF THE INVENTION

### Field of the Invention

The present invention relates to a fusion protein comprising a coronavirus-derived receptor-binding domain and a nucleocapsid protein and the use thereof, and more particularly to the use of the fusion protein for prevention or treatment of coronavirus infection.

### Description of the Related Art

Coronavirus is an RNA virus that contains genetic information in the form of ribonucleic acids (RNA). Coronavirus causes respiratory and digestive system infections in humans and animals. In general, coronavirus is readily infected by mucosal transmission, droplet transmission and the like, and humans usually exhibit mild respiratory infections, but occasionally infection is fatal.

Severe acute respiratory syndrome-coronavirus-2 (SARS-CoV-2) arose in Wuhan, China and spread rapidly around the world. The World Health Organization (WHO) gave the name "COVID-19" to the disease caused by the virus. In accordance with the spread of SARS-CoV-2 infection, the WHO declared a "Public Health Emergency of International Concern" (PHEIC) on January 30. Because the number of confirmed COVID-19 cases continued to increase all over the world, the WHO declared a global pandemic for the third time in history, following the Hong Kong Flu on March 11 (1968) and the Swine Flu (2009).

About 100 million people have been infected worldwide, about 2 million people have died, tens of thousands to hundreds of thousands of confirmed patients are added every day, and the number of infected patients continues to increase (As of January 18, 2021, COVID-19 Dashboard by the Center for Systems Science and Engineering (CSSE) at Johns Hopkins University (JHU)). In Korea, about 70,000 patients were confirmed, and hundreds of confirmed cases are added every day due to the spread by infection through local communities, and a total of 1,264 deaths have been reported (as of January 18, 2021, Central Disaster and Safety Countermeasures Headquarters in Korea).

Infection with SARS-CoV-2 is known to cause fever (83-99%), cough (59-82%), loss of appetite (40-84%), fatigue (44-70%), dyspnea (31-40%), phlegm and cough (28-33%), muscle pain and other pain (11-35%) and the like (US Centers for Disease Control and Prevention (CDC). 6 April 2020), and these symptoms were reported to develop after an incubation period of 2 to 14 days.

SARS-CoV-2 is highly contagious, is known to be generally transmitted through direct/indirect contact or aerosol-type droplets discharged from infected persons, and is also reported to often be transmitted through asymptomatic patients (European Centre for Disease Prevention and Control. Retrieved 30 April 2020). In addition, cases of suffering from sequelae or reinfection even after complete recovery have been repeatedly reported. Therefore, the prevention of SARS-CoV-2 infection is of utmost importance. For this purpose, masks, hand washing and social distancing are actively performed at the personal/social levels, but cases of SARS-CoV-2 infection occur at various sites comprising churches, and the infection route thereof is unclear. Thus, there is urgent demand for the development of SARS-CoV-2 vaccines. Vaccine candidates are currently being developed and researched by various institutions, and various types of vaccines such as recombinant vaccines, inactive viral vaccines and mRNA vaccines are in clinical trials. Recently, vaccines developed by some pharmaceutical manufacturers such as Pfizer, Moderna, and Astrazeneca have been urgently approved and released, but production and supply are extremely insufficient to meet the demand, and moreover, multiple variants of coronavirus are sequentially arising. Accordingly, there is continuous need for the development of novel vaccines.

Meanwhile, angiotensin-converting enzyme 2 (ACE2) is known to protect the kidneys, lungs and heart from inflammation by degrading angiotensin II, which is a pro-inflammatory substance. ACE2 is known to play an important role as a viral receptor in the cell entry process of the coronavirus family such as SARS-CoV-1 and SARS-CoV-2. Specifically, SARS-CoV-2 has been reported to act through two mechanisms, namely direct fusion due to co-action with TMPRSS2 and intracellular action based on the binding of ACE 2 and the spike protein (S protein) (Int. J. Mol. Sci. 2020, 21, 5224; 10.1016/j.cell.2020.02.052 et al.). Therefore, strategies for developing many therapeutic agents and vaccines that are currently being researched target spike proteins, and in particular, most vaccines aim to form neutralizing antibodies against the spike proteins of SARS-CoV-2.

Most recombinant vaccines that are introduced only with the antigenic determinant site of the pathogen separately produced using the genetic information of the pathogen have an antibody-mediated virus-neutralizing effect through the formation of a neutralizing antibody against spike protein or RBD as a pharmacological mechanism by administering spike proteins or fragments thereof, particularly, the receptor-binding domain (RBD) which bind to ACE2, as an antigen.

In particular, the SARS-CoV-2 receptor-binding domain (RBD) is an amino acid of a specific sequence that binds to ACE2, which is a receptor comprised in the spike protein (S protein) of SARS-CoV-2, the wild-type amino acid sequence (SEQ ID NO: 1) thereof has been reported, and the results of research on the binding structure thereof have been continuously reported (Nature. 30 March; Science. 3 April; Cell. 9 April).

Under this background art, as a result of extensive efforts to develop vaccines for preventing or treating infections with coronaviruses comprising COVID-19, the present inventors prepared a fusion protein comprising a receptor-binding domain of SARS-CoV-2-derived S protein, a nucleocapsid protein RNA-binding domain and an Fc domain, and found that an animal model vaccinated with the fusion protein exhibits a remarkably high receptor-binding-domain-specific neutralizing antibody titer and activated T-cell immune response compared to a fusion protein comprising only the receptor-binding domain. Based on this finding, the present invention has been completed.

The information disclosed in this Background section is provided only for better understanding of the background of the present invention, and therefore it may not comprise information that forms the prior art that is already obvious to those skilled in the art.

### SUMMARY OF THE INVENTION

Therefore, the present invention has been made in view of the above problems, and it is one object of the present invention to provide a fusion protein having coronavirus-specific immunogenicity and the use thereof.

It is another object of the present invention to provide a composition for preventing or treating coronavirus infection containing the fusion protein.

It is another object of the present invention to provide a vaccination method for preventing or treating coronavirus infection.

It is another object of the present invention to provide a method for preventing or treating coronavirus infection.

It is another object of the present invention to provide a nucleic acid encoding the fusion protein.

It is another object of the present invention to provide a recombinant vector comprising the nucleic acid.

It is another object of the present invention to provide a host cell into which the nucleic acid or recombinant vector is introduced.

It is another object of the present invention to provide a method for producing a fusion protein.

In accordance with one aspect of the present invention, the above and other objects can be accomplished by the provision of a fusion protein comprising a SARS-CoV-2-derived S protein receptor-binding domain, a nucleocapsid protein and an Fc domain.

In accordance with another aspect, the present invention provides a vaccine composition comprising the fusion protein.

In accordance with another aspect, the present invention provides the use of the fusion protein for the manufacture of a vaccine composition for preventing coronavirus infection.

In accordance with another aspect, the present invention provides a method for vaccination against coronavirus infection, the method comprising administering the fusion protein and/or vaccine composition to a subject.

In accordance with another aspect, the present invention provides a pharmaceutical composition for preventing or treating coronavirus infection comprising the fusion protein as an active ingredient.

In accordance with another aspect, the present invention provides the use of the fusion protein for the manufacture of a pharmaceutical composition for preventing or treating coronavirus infection.

In accordance with another aspect, the present invention provides a method for preventing or treating coronavirus infection, the method comprising administering the fusion protein and/or pharmaceutical composition to a subject.

In accordance with another aspect, the present invention provides the use of the fusion protein for the prevention or treatment of coronavirus infection.

In accordance with another aspect, the present invention provides a nucleic acid encoding the fusion protein.

In accordance with another aspect, the present invention provides a recombinant vector comprising the nucleic acid.

In accordance with another aspect, the present invention provides a host cell into which the nucleic acid or recombinant vector is introduced.

In accordance with another aspect, the present invention provides a method for producing a fusion protein, the method comprising culturing the host cell.

### BRIEF DESCRIPTION OF THE DRAWINGS

The above and other objects, features and other advantages of the present invention will be more clearly understood from the following detailed description taken in conjunction with the accompanying drawings, in which:
FIG. 1 shows a binding structure between a SARS-CoV-2 receptor-binding domain (RBD) and ACE2 (Nature. 30 March);
FIG. 2 shows the amino acid sequence of wild-type RBD, wherein each shaded part indicates a receptor-binding motif (RBM) and the blue boxes indicate epitopes of MHC I and MHC II;
FIG. 3 is a schematic diagram showing the fusion protein prepared in Example;
FIG. 4 is a schematic diagram showing IgG1-Fc (left), and shows the results of SDS-PAGE on the product (right).
FIG. 5 is a schematic diagram showing Fc-RBDwt (left) and shows the results of SDS-PAGE on the product (right);
FIG. 6 is a schematic diagram of RBDwt-Fc (left) and shows the results of SDS-PAGE on the product (right);
FIG. 7 is a schematic diagram showing N-Fc (left) and shows the results of SDS-PAGE on the product (right);
FIG. 8 is a schematic diagram of GIC-1114 (left) and shows the results of SDS-PAGE on the product (right);
FIG. 9 is a schematic diagram showing GIC-1114N;
FIG. 10 is a schematic diagram showing vaccination of a mouse animal model with the fusion protein of the present invention;
FIG. 11 shows the titer of RBD-specific neutralizing antibody formation in a mouse animal model vaccinated with each of IgG Fc, GIC-1114 and Fc-RBDwt fusion proteins alone or a combination thereof with an adjuvant.
FIG. 12 shows IFN-γ levels of T cells in the spleen of mice vaccinated with GIC-1114, Fc-RBDwt and N-Fc fusion proteins when stimulated with each of the indicated antigens.

### DETAILED DESCRIPTION OF THE INVENTION

Hereinafter, the present invention will be described in detail. However, the following detailed description is provided only for illustration of the present invention, and should not be construed as limiting the scope of the present invention. A variety of modifications and alterations are possible without departing from the scope of the claims set forth later and equivalents thereto.

Unless otherwise indicated, nucleic acids and amino acids are written from left to right, in the 5' to 3' direction, and in the N-terminus to C-terminus direction. Numerical ranges listed herein comprise the numbers defining the range, and comprise any integer or non-integer fraction within the defined range.

Unless defined otherwise, all technical and scientific terms used herein have the same meanings as appreciated by those skilled in the field to which the present invention pertains. While any methods and materials similar or equivalent to those described herein may be used in practice to test the present invention, preferred materials and methods are described herein.

SARS-CoV-2, which was first found in China in December 2019, is a pandemic with tens of millions of confirmed cases and hundreds of thousands of deaths all over the world. Despite these circumstances, there are still few therapeutic agents and vaccines for SARS-CoV-2 that have proven to be effective, and many researchers and companies are making great efforts to develop therapeutic agents and vaccines therefor.

Most SARS-CoV-2 vaccines currently being developed aim to produce neutralizing antibodies using, as immunogens, spike proteins or fragments thereof, which are reported to play the most important role in the mechanism of infection (cell entry) and symptom expression of SARS-CoV-2.

In an embodiment of the present invention, a fusion protein was produced by fusing a nucleocapsid protein as well as the SARS-CoV-2-derived spike protein receptor-binding domain, with an Fc domain. A fusion protein comprising an Fc domain can exhibit improved biological properties such as prolonged half-life and increased expression levels while minimizing loss of biological activity.

In another embodiment of the present invention, the result of vaccination of the mouse animal model with the fusion protein comprising the receptor-binding domain, the nucleocapsid protein and the Fc domain of the present invention showed that the fusion protein according to the present invention exhibits remarkably higher titer of the SARS-CoV-2 receptor-binding protein-specific neutralizing antibody formation than a fusion protein comprising no nucleocapsid protein and that the mouse spleen-derived T cells vaccinated with the fusion protein according to the present invention exhibit high IFN-γ level when stimulated with the nucleocapsid protein (N-Fc), which means that the fusion protein is capable of activating the T-cell immune response.

In one aspect, the present invention is directed to a fusion protein comprising a SARS-CoV-2-derived S protein receptor-binding domain, a nucleocapsid protein and an Fc domain.

As used herein, the term "receptor-binding domain (RBD)" refers to a specific amino acid sequence that binds to ACE2, which is a receptor of coronavirus spike proteins, and comprises a receptor-binding motif (RBM). Preferably, the receptor-binding domain may be a domain of a spike protein derived from SARS-CoV-2. For example, the wild-type amino acid sequence (SEQ ID NO: 1) of the SARS-CoV-2-derived receptor-binding domain has been reported (Nature. 30 March).

As used herein, the term "receptor-binding motif (RBM)" is an amino acid sequence comprised in the receptor-binding domain (RBD), and has a site contacting the receptor ACE2. For example, the wild-type amino acid sequence (120-188 amino acids of SEQ ID NO: 1) of the SARS-CoV-2-derived receptor-binding motif has been reported (Nature. 30 March).

A coronavirus spike protein is known to play a very important role not only in cell infection with coronavirus, but also in virus reassembly and release. In particular, the receptor-binding domain is a domain that is directly involved in binding and interaction with ACE2, which is a receptor of the spike protein, and is characterized in that the sequence thereof is highly conserved among coronavirus species.

In the present invention, the coronavirus receptor-binding domain or receptor-binding motif may be the receptor-binding domain or motif of the spike protein of currently reported coronavirus or the receptor-binding domain or motif of the spike protein of variant coronavirus having a gene mutation.

In the present invention, the receptor-binding domain may be derived from SARS-CoV-2, but is not limited thereto.

In the present invention, the receptor-binding domain may be represented by the amino acid sequence of SEQ ID NO: 1, but is not limited thereto. The receptor-binding domain may have a sequence having high homology with the amino acid sequence of SEQ ID NO: 1, for example, a sequence having high homology of 80% or more, 90% or more, preferably 95% or more, and most preferably 99% or more.

As used herein, the term "nucleocapsid protein" refers to a genetic material of a virus and a capsid protein surrounding RNA for coronavirus. Expression of nucleocapsid proteins has been reported to be remarkably increased in coronavirus-infected cells, and in particular, the RNA-binding domain of the nucleocapsid protein is a highly conserved sequence. In the present invention, the term "nucleocapsid protein" is used interchangeably with "N protein", having the same meaning as above.

In the present invention, the nucleocapsid protein is intended to encompass not only the entire nucleocapsid protein, but also a specific domain or fragment of the nucleocapsid protein, and preferably comprises a RNA-binding domain of the nucleocapsid protein or a fragment comprising the same.

In the present invention, the N protein may be represented by the sequence of SEQ ID NO: 3, or may comprise a sequence represented by SEQ ID NO: 3.

In the present invention, the fusion protein may comprise the RNA-binding domain of the N protein.

In the present invention, the RNA-binding domain may be represented by SEQ ID NO: 4.

As used herein, the term "Fc domain" refers to a tail region of an antibody that interacts with the receptor on the cell surface and with proteins of the complement system in immunoglobulins, comprises the heavy-chain constant domains CH2 and CH3, and may further comprise hinge regions of the heavy-chain constant domains. In the present invention, the Fc domain is intended to encompass all of the Fc domains of an immunoglobulin, a fragment thereof, and a variant thereof.

In the present invention, the Fc domain may be a mammalian immunoglobulin Fc domain, preferably a mouse, rabbit or human immunoglobulin Fc domain, and more preferably a human immunoglobulin Fc domain, but is not limited thereto.

In the present invention, the Fc domain may be an IgA, IgM, IgE, IgD or IgG Fc domain, a fragment thereof, or a variant thereof, and preferably, the Fc domain is an IgG Fc domain (e.g. an IgG1, IgG2a, IgG2b, IgG3 or IgG4 Fc domain), but is not limited thereto.

In the present invention, the Fc domain may be a human IgG1 Fc domain. More specifically, the Fc domain may be an Fc domain derived from IgG1, IgG2a, IgG2b, IgG3 or IgG4, which is an IgG isotype derived from an organism.

In the present invention, the Fc domain is most preferably represented by the sequence of SEQ ID NO: 2, or comprises the sequence, but is not limited thereto.

In addition, the Fc domain may comprise a sugar chain, or may take a sugar chain that is increased or decreased compared to the native form, or a form in which a sugar chain is removed, compared to the native form. The increase, decrease, or removal of the sugar chain may be performed by a conventional method known in the art such as a chemical method, an enzymatic method, and a genetic-engineering method using microorganisms. Herein, the removal of the sugar chain from the Fc domain remarkably reduces binding affinity for C1q of the primary complement component, C1, and causes reduction or loss of antibody-dependent cell-mediated cytotoxicity (ADCC) or complement-dependent cytotoxicity (CDC), thus preventing an undesired immune response *in vivo.*

In the present invention, the fusion protein may comprise two or more proteins comprising a receptor-binding domain, a nucleocapsid protein and an Fc domain.

In the present invention, the fusion protein may be a dimer of a protein comprising a receptor-binding domain, a nucleocapsid protein and an Fc domain.

In the present invention, the dimer may be formed through disulfide bonds of one or more cysteine residues of each Fc domain.

In the present invention, the fusion protein may be a heterodimer or a homodimer of a protein comprising a receptor-binding domain, a nucleocapsid protein, and an Fc domain. In one embodiment of the present invention, the fusion protein is prepared as a homodimer, but is not limited thereto.

Amino acid sequences of the receptor-binding domain, nucleocapsid protein, Fc domain and the like mentioned herein are to be interpreted as comprising variants or fragments thereof in which amino acid residues are conservatively substituted at specific amino acid residue positions.

As used herein, the term "conservative substitution" refers to modification comprising substitution of one or more amino acids with amino acids having similar biochemical properties without causing loss of the biological or biochemical function of the corresponding protein.

The term "conservative amino acid substitution" refers to substitution of amino acid residues with other amino acid residues having side chains similar thereto. For example, classes of amino acid residues having similar side chains are well known in the art. These classes comprise amino acids having basic side chains (e.g. lysine, arginine, histidine), amino acids having acidic side chains (e.g. aspartic acid, glutamic acid), amino acids having uncharged polar side chains (e.g. glycine, asparagine, glutamine, serine, threonine, tyrosine, cysteine), amino acids having non-polar side chains (e.g., alanine, valine, leucine, isoleucine, proline, phenylalanine, methionine, tryptophan), amino acids having beta-branched side chains (e.g., threonine, valine, isoleucine), and amino acids having aromatic side chains (e.g., tyrosine, phenylalanine, tryptophan, histidine).

In the present invention, the receptor-binding domain binds to ACE2 based on a receptor-binding motif. Therefore, in the present invention, it is preferred that conservative substitutions occur in portions of the amino acid sequence other than the receptor-binding domain and motif, but the invention is not limited thereto.

In the present invention, components such as the receptor-binding domain, the Fc domain and the nucleocapsid protein comprised in the fusion protein may be directly linked to one another through covalent bonds or the like, and may be indirectly linked to one another via other molecules, for example, linkers.

In the present invention, the receptor-binding domain, the nucleocapsid protein and the Fc domain are preferably linked to one another via a glycine-serine linker (GS linker), more preferably a G4S linker. In the present invention, preferably, the receptor-binding domain, the nucleocapsid protein and the Fc domain are preferably linked to one another via a linker comprising the amino acid sequence of SEQ ID NO: 5 and/or SEQ ID NO: 6.

In one embodiment of the present invention, a fusion protein was prepared by linking a receptor-binding domain (or a variant thereof) or a N protein derived from SARS-CoV-2 to the C-terminus and/or N-terminus of the Fc domain using a linker represented by GGGGSGGGGSGGGGS (SEQ ID NO: 5) or GGGGSGGGGSGGGGSGGGGS (SEQ ID NO: 6), but the present invention is not limited thereto.

In the present invention, preferably, the fusion protein may have a structure in which the receptor-binding domain and the coronavirus-derived nucleocapsid protein are linked to the Fc domain.

In the present invention, the receptor-binding domain may be linked to the N-terminus and/or C-terminus of the Fc domain, and preferably to the C-terminus thereof.

In the present invention, the receptor-binding domain may be linked to one of the N-terminus or C-terminus of the Fc domain, and the nucleocapsid protein (N protein) may be linked to a remaining one thereof.

In the present invention, preferably, the receptor-binding domain may be linked to the C-terminus of the Fc domain, and the nucleocapsid protein may be linked to the N-terminus of the Fc domain.

In the present invention, the receptor-binding domain is linked to one of the N-terminus and C-terminus of the Fc domain, and the RNA-binding domain of the nucleocapsid protein may be linked to a remaining one thereof.

In the present invention, the fusion protein may have a structure in which the receptor-binding domain, the Fc domain and the nucleocapsid protein are linked in that order or in which the nucleocapsid protein, the Fc domain and the receptor-binding domain are linked in that order.

In the present invention, the fusion protein is preferably represented by the amino acid sequence of SEQ ID NO: 19 or SEQ ID NO: 20, or comprises the same.

In the present invention, the fusion protein may be a dimer of an Fc domain to which the receptor-binding domain and the nucleocapsid protein are linked.

In the present invention, the fusion protein is a homodimer or heterodimer of a "receptor-binding domain-Fc domain-nucleocapsid protein" and/or a "nucleocapsid protein-Fc domain-receptor-binding domain".

The expressions "receptor-binding domain-Fc domain-nucleocapsid protein" and "nucleocapsid protein-Fc domain-receptor-binding domain" refer to proteins in which respective components (receptor-binding domain, Fc domain and nucleocapsid protein) are sequentially linked from the C-terminus to the N-terminus.

In the present invention, the fusion protein is preferably a homodimer or heterodimer of a protein that is represented by the amino acid sequence of SEQ ID NO: 19 or SEQ ID NO: 20, or comprises the same.

In the present invention, the fusion protein is most preferably a homodimer of a protein represented by SEQ ID NO: 19 or SEQ ID NO: 20.

In the present invention, the receptor-binding domain and the nucleocapsid protein may be directly linked to the Fc domain through a covalent bond, and may be indirectly linked via a linker such as a linker, protein, or polysaccharide.

In the present invention, vaccination with the fusion protein is capable of inducing a remarkable improvement in the titer of the mouse neutralizing antibody formation and T-cell immune response, preventing viral infection, inhibiting proliferation of the virus and eliminating the virus.

As used herein, the term "coronavirus" refers to an RNA virus that belongs to the genus *Coronavirinae* (Ninth Report of the International Committee on Taxonomy of Viruses. Elsevier, Oxford. pp. 806-828). The genus *Coronavirinae* is divided into four genera, namely alpha, beta, gamma, and delta coronavirus genera. Examples of coronaviruses that may infect humans comprise SARS-CoV, MERS-CoV, SARS-CoV-2, HCoV-229E, HCoV-OC43, HKU1, HCoV-NL63, and the like, but are not limited thereto.

In one embodiment of the present invention, a protein fused with a SARS-CoV-2-derived receptor-binding domain and an RNA-binding domain was produced, but all coronaviruses have a spike protein in common, and enters and proliferates into the host cell through binding to ACE2, which is a receptor in the host cell thereof. In particular, it is obvious that the receptor-binding domain comprised in the S1 subunit of each species is highly conserved, and thus the fusion protein of the present invention is not limited to comprising molecules derived from SARS-CoV-2.

In the present invention, the fusion protein may comprise a molecule other than the coronavirus-derived receptor-binding domain, the nucleocapsid protein and the Fc domain, and may further, for example, comprise a coronavirus-derived molecule (preferably a SARS-CoV-2-derived molecule), linker, adjuvant, antigen, antibody or the like, but is not limited thereto.

In the present invention, the coronavirus-derived substance means any substance derived from coronavirus, such as a coronavirus-derived protein, nucleic acid (preferably RNA) or polysaccharide.

In the present invention, the coronavirus-derived substance may refer to any substance produced by or contained in coronavirus, such as a protein, nucleic acid or polysaccharide isolated from the coronavirus, for example, an N protein, M protein, ORF protein, or the like. For example, the fusion protein of the present invention may further comprise an M protein, but is not limited thereto.

In the present invention, the coronavirus-derived substance may be a coronavirus-derived protein or a fragment thereof.

In one embodiment of the present invention, it was found that a fusion protein prepared by linking a receptor-binding domain to one terminus (N-terminus or C-terminus) of the Fc domain and linking an RNA-binding domain of a nucleocapsid protein to the other terminus thereof has a high titer of the neutralizing antibody formation and T-cell immune response induction ability.

The fusion protein of the present invention exhibits immunogenicity and thus can induce various immune responses in the body, and can, for example, induce the formation of neutralizing antibodies, stimulate the differentiation of cytotoxic T lymphocytes, and stimulate the differentiation of Th cells. In one embodiment of the present invention, it was found that the fusion protein of the present invention can induce remarkably higher titer of the neutralizing antibody formation and better T-cell immune response than a fusion protein comprising a receptor-binding domain and an Fc-domain.

Accordingly, in another aspect, the present invention is directed to a vaccine composition comprising the fusion protein of the present invention.

As used herein, the term "vaccine composition" refers to a composition comprising a substance that acts as an antigen or immunogen *in vivo* or *in vitro* to induce an immune response, and may be used interchangeably with "vaccine" or "immunogenic composition" having the same meaning as above.

As used herein, the term "immune response" is intended to encompass both innate and adaptive immune responses, for example, complement-mediated immune responses, cell (T cell)-mediated immune responses, and/or antibody (B cell) responses.

The vaccine composition of the present invention can induce or improve an immune response against coronavirus in a subject to which the vaccine composition is administered, and more specifically, can induce formation of neutralizing antibodies against SARS-CoV-2 virus, induce and/or increase differentiation of cytotoxic lymphocytes, prevent, ameliorate, eliminate or reduce the likelihood of reactivation of the virus, and/or prevent or reduce the likelihood of the onset of other diseases or complications associated with reactivation of the virus, when the subject is infected with SARS-CoV-2.

In the present invention, the vaccine composition may further comprise an adjuvant.

As used herein, the term "adjuvant" refers to a concept based on the discovery by Alexander Glenny that an aluminum salt increases the immune response, and refers to an auxiliary component added to induce a stronger immune response in a subject to which a vaccine composition or vaccine is administered. Examples of the adjuvant may comprise aluminum salts such as aluminum phosphate or aluminum hydroxide, squalene-containing emulsions such as MF59 or analogues thereof (MF59-like substances), AS03 or analogues thereof (AS03-like substances), AF03 or analogues thereof (AF03-like substances), and SE or analogues thereof (SE-like substances), calcium salts, dsRNA analogues, lipopolysaccharides, lipid A analogues (MPL-A, GLA, etc.), flagellins, imidazoquinolines, CpG ODN, mineral oils, Toll-like receptor (TLR) antagonists, C-type lectin ligands, CD1d ligand (α-galactosylceramide, etc.), detergents, liposomes, saponins such as QS21, cytokines, peptides, and the like, but are not limited thereto.

In one embodiment of the present invention, as an adjuvant, AddaVax (MF59 like), MPLA, Alum or the like was used alone or in combination, and it was confirmed that the immune response was boosted in all cases.

Therefore, in the present invention, the adjuvant is preferably selected from the group consisting of MF59, Alum, MPLA and combinations thereof, and is more preferably MF59 alone, MPLA alone, or a combination of Alum and MPLA, but is not limited thereto.

The optimal dosage of the vaccine composition of the present invention can be determined by standard research involving observation of a suitable immune response in a subject. After initial vaccination, the subject may be subjected to one or more booster immunizations at appropriate intervals. In one embodiment of the present invention, 2 weeks after the initial vaccination in the mouse animal model, additional vaccination was performed, but is not limited thereto.

The vaccine composition of the present invention may be administered in a pharmaceutically effective amount, and the term "pharmaceutically effective amount" refers to an amount sufficient to induce or increase an immune response, without causing side effects or serious or excessive immune responses. The suitable dosage may vary depending on a variety of factors comprising the formulation method, mode of administration, the age, weight, gender, and pathological condition of the patient, diet, administration time, route of administration, excretion rate and response sensitivity. Various general considerations when determining a pharmaceutically effective amount are known to those of skill in the art, and are set forth in references such as [Gilman et al., eds., Goodman And Gilman's: The Pharmacological Bases of Therapeutics, 8th ed., Pergamon Press, 1990] and [Remington's Pharmaceutical Sciences, 17th ed., Mack Publishing Co., Easton, Pa., 1990].

The vaccine composition of the present invention may be administered in combination with a therapeutic agent for coronavirus or symptomatic therapeutic agent, and may be administered in combination with other vaccines, virus therapeutic agents, immune adjuvants, symptom relief agents, and the like.

The vaccine composition of the present invention may be prepared into a unit dosage form, or may be incorporated into a multi-dose container by formulating the same using a pharmaceutically acceptable carrier and/or excipient according to a method that can be easily implemented by a person having ordinary skill in the art to which the present invention pertains. The formulation may be prepared and used in the form of oral formulations such as powders, granules, tablets, capsules, suspensions, emulsions, syrups and aerosols, external preparations, suppositories and sterilized injection solutions according to a conventional method. Suitable formulations known in the art may be those disclosed in the reference Remington's Pharmaceutical Science (Mack Publishing Company, Easton PA). Solid formulations for oral administration comprise tablets, pills, powders, granules, capsules and the like. Such solid formulations are prepared by mixing at least one excipient such as starch, calcium carbonate, sucrose, lactose, gelatin and the like. In addition to simple excipients, lubricants such as magnesium stearate and talc are also used. Liquid formulations for oral administration comprise suspensions, oral liquids and solutions, emulsions, syrups and the like. Various excipients such as wetting agents, sweeteners, fragrances, preservatives and the like may be comprised, in addition to water and liquid paraffin, which are simple diluents that are commonly used. Formulations for parenteral administration comprise sterilized aqueous solutions, non-aqueous solutions, suspensions, emulsions, freeze-dried preparations, and suppositories. Examples of a suppository base comprise Witepsol, macrogol, Tween 61, cacao butter, laurin butter, glycerogelatin and the like.

The vaccine composition of the present invention may be administered orally or parenterally. The route of administration of the composition according to the present invention is, for example, intrapulmonary, intravenous, subcutaneous, intramuscular, intraarterial, intramedullary, intrathecal, intracardiac, transdermal, intraperitoneal, intestinal, sublingual, oral or topical administration. The dosage of the composition according to the present invention varies depending on the patent's weight, age, gender, health status, diet, administration time, administration method, excretion rate, or severity of disease, and is easily determined by those skilled in the art. In addition, the composition of the present invention may be prepared into a suitable formulation using known techniques for clinical administration.

In another aspect, the present invention is directed to the use of the fusion protein for the manufacture of a vaccine composition for preventing coronavirus infection.

In another aspect, the present invention is directed to the use of the fusion protein for the manufacture of a vaccine composition for preventing or treating coronavirus infection.

In another aspect, the present invention is directed to a method for vaccination against coronavirus infection, the method comprising administering the fusion protein and/or the vaccine composition to a subject.

In an embodiment of the present invention, a receptor-binding domain derived from SARS-CoV-2 was used, but a virus such as SARS-CoV-1 can be also used for the prevention or treatment of all coronavirus infections since it enters cells through a similar mechanism through the binding of ACE2 and spike protein having a conserved sequence (RBM) and is exhibited as a disease.

In another aspect, the present invention is directed to a pharmaceutical composition for preventing or treating coronavirus infection comprising the fusion protein as an active ingredient.

As used herein, the term "prevention" refers to any action causing the suppression or delay of the onset of a disease of interest by administering the pharmaceutical composition according to the present invention.

As used herein, the term "treatment" refers to any action causing an improvement in symptoms of a disease of interest or the beneficial alteration of the symptoms by administering the pharmaceutical composition according to the present invention.

The pharmaceutical composition according to the present invention may further comprise an appropriate carrier, excipient or diluent typically used for pharmaceutical compositions.

Specifically, the carrier, excipient or diluent that may be comprised in the pharmaceutical composition may comprise lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil. The composition may be formulated using a commonly used diluent or excipient such as a filler, an extender, a binder, a wetting agent, a disintegrant or a surfactant.

The pharmaceutical composition according to the present invention may be used in various formulations according to a conventional method. Suitable formulations comprise oral formulations such as tablets, pills, powders, granules, Sugar Coat tablet, hard or soft capsules, solutions, suspensions or emulsions, injections and aerosols, external preparations, suppositories and sterile injectable solutions, but are not limited thereto.

The pharmaceutical composition according to the present invention may be prepared in a suitable formulation using a pharmaceutically inert organic or inorganic carrier. That is, when the formulation is a tablet, a coated tablet, a dragée or a hard capsule, it may comprises lactose, sucrose, starch or a derivative thereof, talc, calcium carbonate, gelatin, stearic acid, or a salt thereof. In addition, when the formulation is a soft capsule, it may comprise a vegetable oil, wax, fat, or semi-solid or liquid polyol. In addition, when the formulation is a solution or syrup, it may comprise water, polyol, glycerol, vegetable oil, or the like.

The pharmaceutical composition according to the present invention may further comprise a preservative, a stabilizer, a wetting agent, an emulsifier, a solubilizing agent, a sweetening agent, a colorant, an osmotic pressure regulator, an antioxidant, or the like, in addition to the carrier.

The pharmaceutical composition of the present invention may be administered in a pharmaceutically effective amount, and the term "pharmaceutically effective amount" refers to an amount sufficient for treating a disease at a reasonable benefit/risk ratio applicable to all medical treatments, and the effective dosage level may vary depending on a variety of factors comprising the type and severity of the disease of the patient, the activity of the drug, the sensitivity of the patient to the drug, the administration time, administration route and excretion rate of the composition according to the present invention, the treatment period, and drugs used concurrently therewith, along with other factors well-known in the pharmaceutical field. The pharmaceutical composition of the present invention may be administered as a single therapeutic agent or in combination with other therapeutic agents, may be administered sequentially or simultaneously with a conventional therapeutic agent, and may be administered in single or multiple doses. Taking into consideration these factors, it is important to administer the minimum amount sufficient to achieve maximum efficacy without side effects, and such an amount can be easily determined by those skilled in the art.

The pharmaceutical composition according to the present invention may be administered orally or parenterally. Parenteral administration may be intravenous injection, subcutaneous injection, intramuscular injection, intraperitoneal injection, endothelial administration, topical administration, intranasal administration, pulmonary administration, rectal administration or the like. Upon oral administration, since proteins or peptides are digested, an oral composition may be coated with an active drug or formulated so as to protect the same from degradation in the stomach. In addition, the composition may be administered using any device capable of delivering the active substance to target cells.

The method of administering the pharmaceutical composition according to the present invention may be easily selected depending on the formulation, and may be administered orally or parenterally. The dosage may vary depending on the patient's age, gender, weight, severity of the disease, and route of administration.

In the present invention, the coronavirus infection is most preferably SARS-CoV-2 infection (COVID-19).

In the present invention, the pharmaceutical composition may be used in combination with other compositions or methods for treating coronavirus infections.

In another aspect, the present invention is directed to the use of the fusion protein for the manufacture of a pharmaceutical composition for preventing or treating coronavirus infection.

In another aspect, the present invention is directed to a method for preventing or treating coronavirus infection, the method comprising administering the fusion protein, the vaccine composition and/or the pharmaceutical composition to a subject.

In another aspect, the present invention is directed to the use of the fusion protein, the vaccine composition and/or the pharmaceutical composition for the prevention or treatment of coronavirus infection.

In another aspect, the present invention is directed to a nucleic acid encoding the fusion protein.

The nucleic acid used herein may be present in a cell or a cell lysate, or may be present in a partially purified form or in a substantially pure form. The nucleic acid may be "isolated" or "become substantially pure" when purified from other cellular components or other contaminants, for example, from nucleic acids or proteins of other cells, by standard techniques comprising, for example, alkaline/SDS treatment, CsCl banding, column chromatography, agarose gel electrophoresis and other techniques well known in the art. The nucleic acid of the present invention may be, for example, DNA or RNA.

In another aspect, the present invention is directed to a recombinant vector comprising the nucleic acid according to the present invention.

Any vector known in the art can be appropriately selected and used as the recombinant vector by those skilled in the art without limitation, so long as it is capable of inducing the expression of a protein encoded by the fusion protein. For example, when *E. coli* is used as a host, vectors comprising T7 series (T7A1, T7A2, T7A3, etc.), lac, lacUV5, temperature-dependent (λphoA), phoB, rmB, tac, trc, trp or 1PL promoters may be used. When yeast is used as a host, vectors comprising the ADH1, AOX1, GAL1, GAL10, PGK or TDH3 promoter may be used, and when *Bacillus* is used as a host, vectors comprising the P2 promoter may be used. These are provided only as representative embodiments, and in addition to the vectors comprising the promoters, any vector can be appropriately selected from various vectors known in the art by those skilled in the art without limitation, so long as it is suitable for a host as a vector comprising a promoter for inducing the expression of the fusion protein according to the present invention.

As used herein, the term "vector" means a DNA product comprising a DNA sequence operably linked to a suitable regulatory sequence capable of expressing the DNA in a suitable host. Vectors may be plasmids, phage particles or simply potential genomic inserts. When transformed into a suitable host, vectors may be replicated or perform functions independent of the host genomes, or some thereof may be integrated with the genomes. A plasmid is currently the most commonly used form of vector, and thus the terms "plasmid" and "vector" are often used interchangeably. However, the present invention encompasses other forms of vectors that are known in the art or have the same functions as those known in the art. Protein expression vectors used in *E. coli* comprise: pET family vectors from Novagen, Inc (USA); pBAD family vectors from Invitrogen Corp. (USA); PHCE or pCOLD vectors from Takara Bio Inc. (Japan); and pACE family vectors from GenoFocus Inc. (South Korea). In *Bacillus subtilis,* a gene of interest can be inserted into a specific part of the genome to realize protein expression, or a pHT-family vector of MoBiTech (Germany) can be used. Even in fungi and yeast, protein expression is possible using genome insertion or self-replicating vectors. A plant protein expression vector using a T-DNA system such as *Agrobacterium tumefaciens* or *Agrobacterium rhizogenes* can be used. Typical expression vectors for expression in mammalian cell cultures are based on, for example, pRK5 (EP 307,247), pSV16B (WO 91/08291), and pVL1392 (Pharmingen).

As used herein, the term "expression control sequence" means a DNA sequence essential for the expression of a coding sequence operably linked to a particular host organism. Such a control sequence comprises promoters for conducting transcription, operator sequences for controlling such transcription, sequences for encoding suitable mRNA ribosome-binding sites, and sequences for controlling the termination of transcription and translation. For example, control sequences suitable for prokaryotes comprise promoters, optionally operator sequences, and ribosome-binding sites. Control sequences suitable for eukaryotic cells comprise promoters, polyadenylation signals, and enhancers. The factor that has the greatest impact on the expression level of a gene in a plasmid is the promoter. SRα promoters, *cytomegalovirus*-derived promoters and the like are preferably used as promoters for high expression.

Any of a wide variety of expression control sequences may be used for the vector in order to express the DNA sequences of the present invention. Useful expression control sequences comprise, for example, early and late promoters of SV40 or adenovirus, the lac system, the trp system, the TAC or TRC system, T3 and T7 promoters, the major operator and promoter regions of phage lambda, control regions of fd code proteins, promoters of 3-phosphoglycerate kinase or other glycol lyases, promoters of phosphatase, such as Pho5, promoters of yeast alpha-mating systems, and other sequences having configurations and induction activity known to control gene expression of prokaryotic or eukaryotic cells or viruses and various combinations thereof. The T7 RNA polymerase promoter Φ may be useful for expressing proteins in *E. coli.*

When a nucleic acid sequence is "operably linked" when it is placed in a functional relationship with another nucleic acid sequence. This may be gene(s) and control sequence(s) linked in such a way so as to enable gene expression when a suitable molecule (e.g., a transcriptional activator protein) is linked to the control sequence(s). For example, DNA for a pre-sequence or secretory leader is operably linked to DNA for a polypeptide when expressed as a pre-protein involved in the secretion of the polypeptide; a promoter or enhancer is operably linked to a coding sequence when it affects the transcription of the sequence; a ribosome-binding site is operably linked to a coding sequence when it affects the transcription of the sequence; or a ribosome-binding site is operably linked to a coding sequence when positioned to facilitate translation. Generally, the term "operably linked" means that the linked DNA sequence is in contact therewith, and that a secretory leader is in contact therewith and is present in the reading frame. However, the enhancer need not be in contact therewith. The linkage of these sequences is carried out by ligation (linkage) at convenient restriction enzyme sites. When no such site exists, a synthetic oligonucleotide adapter or a linker according to a conventional method is used.

As used herein, the term "expression vector" commonly refers to a recombinant carrier into which a fragment of heterologous DNA is inserted, and generally means a fragment of double-stranded DNA. Herein, "heterologous DNA" means xenogenous DNA that is not naturally found in the host cell. Once an expression vector is present in a host cell, it can replicate independently of the host chromosomal DNA, and several copies of the vector and inserted (heterologous) DNA thereof can be produced.

As is well known in the art, in order to increase the expression level of a transfected gene in a recombinant cell, the gene should be operably linked to a transcriptional or translational expression control sequence that functions in the selected expression host. Preferably, the expression control sequence and the corresponding gene are comprised in a single expression vector comprising both a bacterial selection marker and a replication origin. When the expression host is a eukaryotic cell, the expression vector should further comprise a useful expression marker in the eukaryotic expression host.

In another aspect, the present invention is directed to a host cell into which the nucleic acid encoding the fusion protein, or the recombinant vector is introduced.

As used herein, the term "host cell" refers to an expression cell introduced with a gene or a recombinant vector to produce a protein or the like. The host cell may be used without limitation, as long as it is a cell capable of expressing the fusion protein of the present invention, and the host cell is preferably a eukaryotic cell, more preferably yeast, an insect cell, or an animal cell, and most preferably an animal cell. For example, the host cell is a CHO cell line or a HEK cell line mainly used for expression of a fusion protein, but is not limited thereto.

Various expression host/vector combinations may be used to express the fusion protein of the present invention. Suitable expression vectors for eukaryotic hosts comprise, for example, but are not limited to, expression control sequences derived from SV40, cow papillomavirus, adenovirus, adeno-associated virus, cytomegalovirus and retrovirus. Expression vectors that can be used for bacterial hosts comprise bacterial plasmids obtained from *Escherichia coli (E. coli),* such as pBluescript, pGEX2T, pUC vectors, col E1, pCR1, pBR322, pMB9 and derivatives thereof, plasmids having a wide host range such as RP4, phage DNA that can be exemplified by a wide variety of phage lambda derivatives such as λ NM989, and other DNA phages such as M13 and filamentous single-stranded DNA phages. Expression vectors useful for yeast cells comprise 2µ plasmids and derivatives thereof. A vector that is useful for insect cells is pVL 941.

The recombinant vector may be introduced into the host cell through transfection or transformation. As used herein, the term "transfection" means introducing DNA into a host and making the DNA replicable using an extrachromosomal factor or chromosomal integration. As used herein, the term "transformation" means that an expression vector is accommodated by the host cell, regardless of whether or not any coding sequence is actually expressed.

It should be understood that not all vectors function identically in expressing the DNA sequences of the present invention. Likewise, not all hosts function identically for the same expression system. However, those skilled in the art will be able to make appropriate selections from among a variety of vectors, expression control sequences and hosts without excessive burden of experimentation and without departing from the scope of the present invention. For example, selection of a vector should be carried out in consideration of a host because the vector should be replicated therein. The number of replications of the vector, the ability to control the number of replications, and the expression of other proteins encoded by the corresponding vector, such as the expression of antibiotic markers, should also be considered. In selecting the expression control sequence, a number of factors should be considered. For example, the relative strength of the sequence, controllability, and compatibility with the DNA sequences of the present invention should be considered, particularly in relation to possible secondary structures. The single-cell host may be selected in consideration of factors such as the selected vector, the toxicity of the product encoded by the DNA sequence of the present invention, secretion characteristics, ability to accurately fold proteins, culture and fermentation factors, and ease of purification of the product encoded by the DNA sequence according to the present invention. Within the scope of these factors, those skilled in the art can select various vector/expression control sequences/host combinations capable of expressing the DNA sequences of the present invention in fermentation or large animal cultures. As a screening method for cloning cDNA of proteins through expression cloning, a binding method, a panning method, a film emulsion method or the like can be applied.

The gene and recombinant vector may be introduced into host cells through various methods known in the art. The gene encoding the nucleic acid encoding the fusion protein of the present invention may be directly introduced into the genome of a host cell and present as a factor on a chromosome. It will be apparent to those skilled in the art to which the present invention pertains that even if the gene is inserted into the genomic chromosome of the host cell, it will have the same effect as when the recombinant vector is introduced into the host cell.

In another aspect, the present invention is directed to a method for producing the fusion protein comprising culturing the host cell.

When a recombinant expression vector capable of expressing the fusion protein is introduced into a mammalian host cell, the fusion protein can be produced by culturing the host cell for a period of time sufficient to allow expression of the fusion protein in the host cell, more preferably, culturing the host cells for a period of time sufficient to allow the fusion protein to be secreted into the culture medium.

In some cases, the expressed fusion protein may be separated from the host cell and purified to homogeneity. The separation or purification of the fusion protein can be carried out using separation and purification methods used for conventional proteins, for example, chromatography. The chromatography may, for example, comprise a combination of one or more selected from affinity chromatography, ion exchange chromatography, and hydrophobic chromatography, but is not limited thereto. A combination of chromatography with filtration, ultrafiltration, salting out, dialysis or the like may be used.

Although specific amino acid sequences and nucleotide sequences are described in the present invention, it will be apparent to those skilled in the art that amino acid sequences substantially identical to the enzymes to be implemented in the present invention and the nucleotide sequences encoding the same fall within the scope of the present invention. The term "substantially identical" comprises the case where the amino acid or nucleotide sequence is highly homologous and the case of a protein that shares structural characteristics regardless of the homology of the sequence or has the same function as that used in the present invention. The present invention may comprise an enzyme from which a portion of a sequence other than the sequence constituting the core of the present invention has been deleted or a fragment of a nucleotide sequence encoding the same, and may comprise all amino-acid or nucleotide sequences having the same function as that used in the present invention regardless of the length of the fragment.

### Example

Hereinafter, the present invention will be described in more detail with reference to the following examples. However, these examples are provided only for illustration of the present invention, and should not be construed as limiting the scope of the present invention.

### Example 1: Preparation of fusion protein

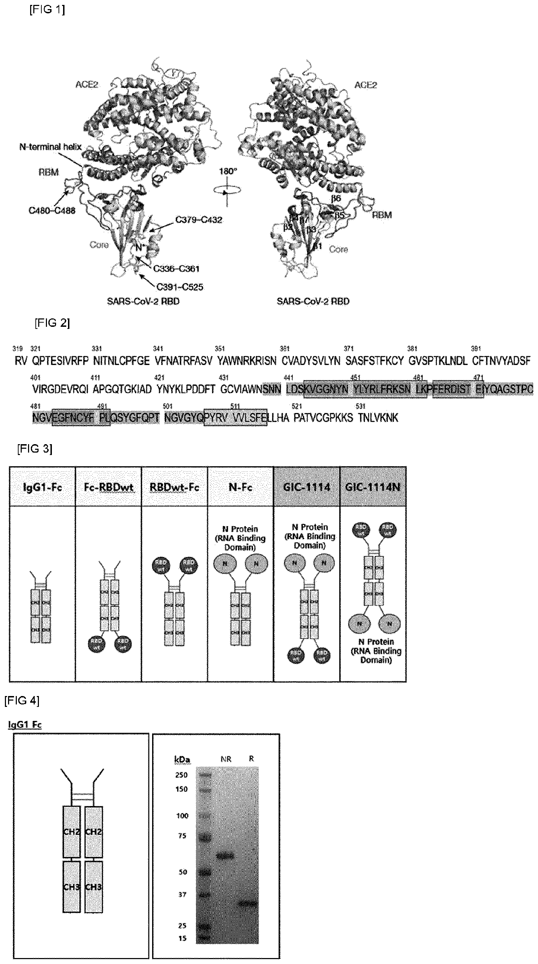

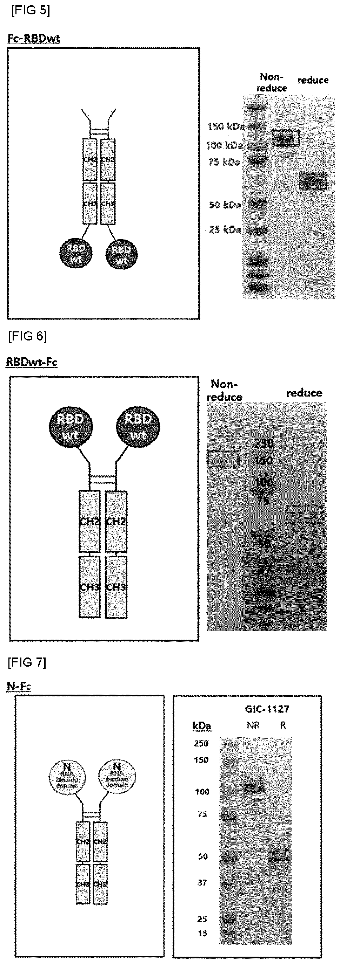

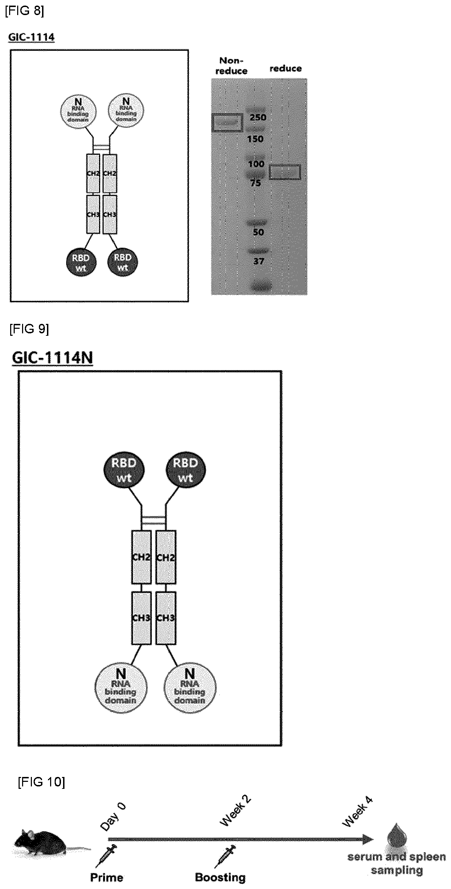

A control group and an experimental group were set as shown in FIG. 3, and a fusion protein (FIGS. 4 to 9) was produced in order to evaluate the ACE2-binding ability and immunogenicity of a fusion protein comprising a receptor-binding domain (RBD), a nucleocapsid protein, and an Fc domain. In order to produce a fusion protein, a nucleotide comprising a base sequence encoding a fusion protein comprising a wild-type RBD protein, a nucleocapsid protein (N protein) RNA-binding domain, an IgG1 Fc domain, linkers, and a signal sequence shown in Table 1 below was synthesized using the gBlock^{™} gene fragments service from Integrated DNA Technologies, and was loaded into pcDNA3.4 vector.

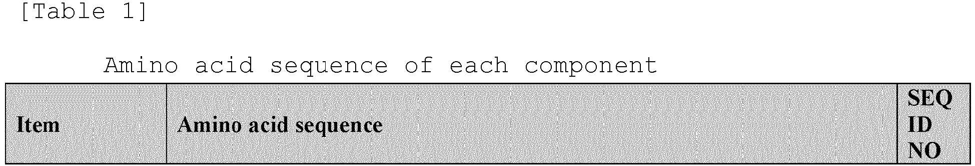

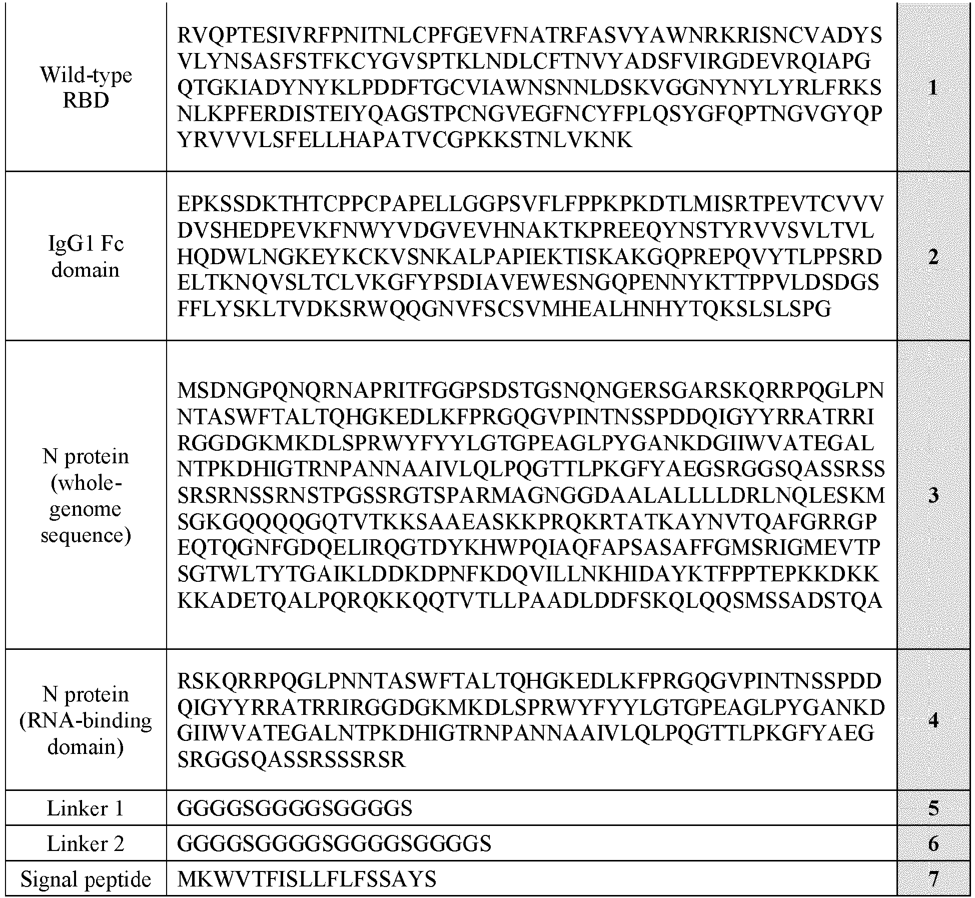

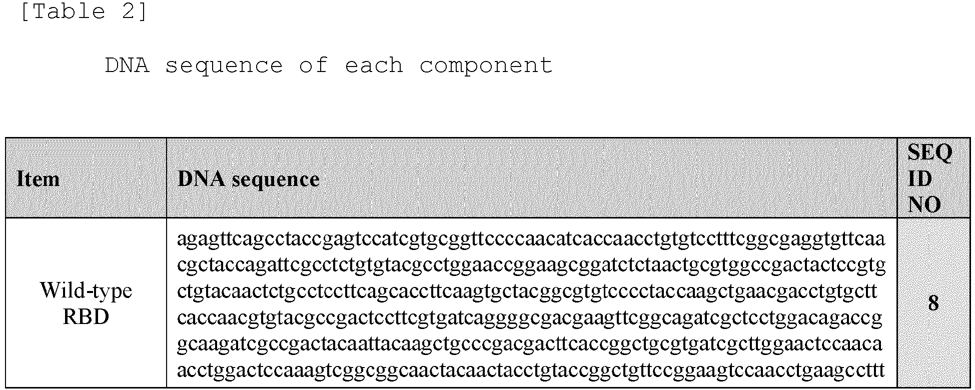

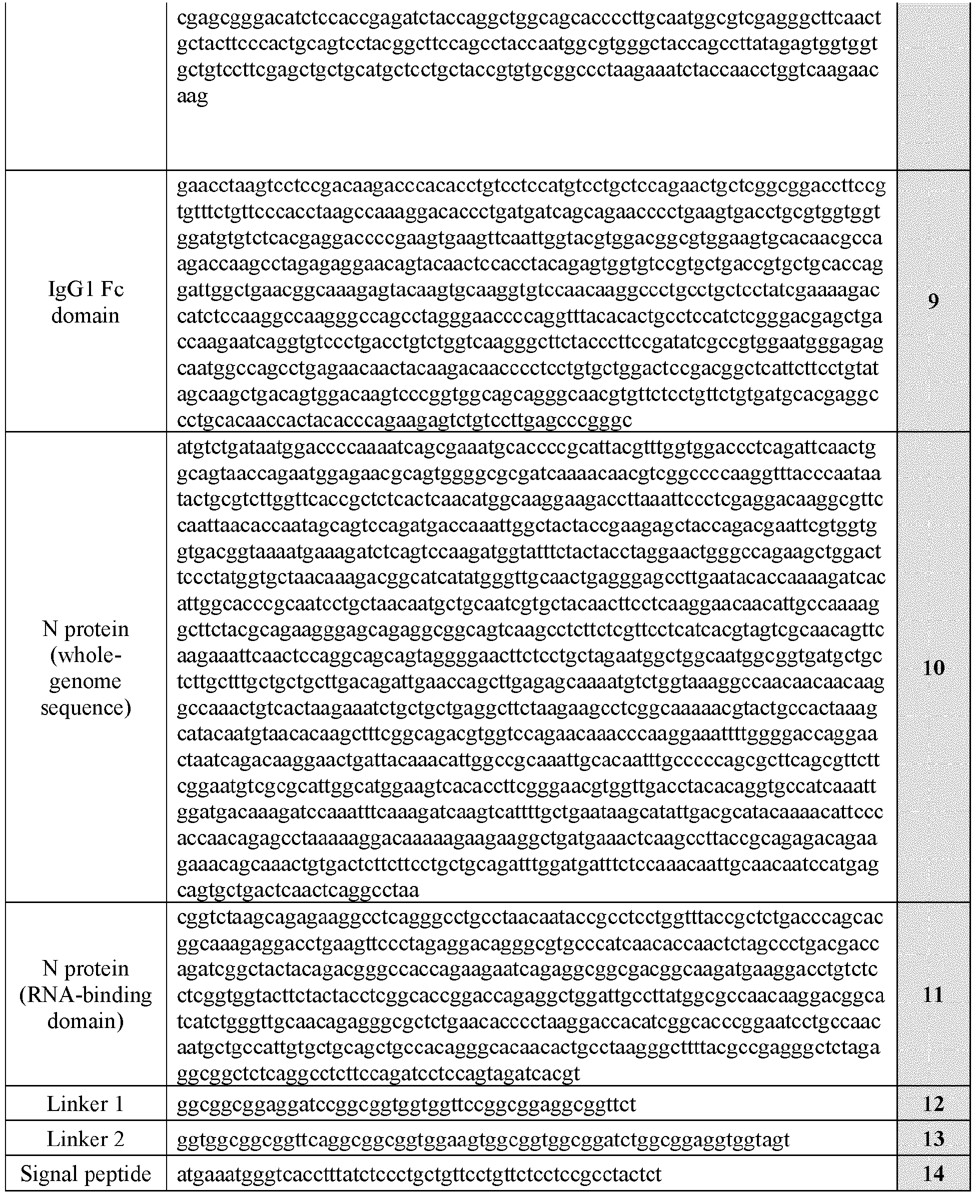

The produced vector was introduced into CHO cells (Expi-CHO^{™}) to express each fusion protein. After introduction of the vector, culture was performed under a 5% CO₂ environment at 37°C and at 125 RPM for 7 days, the culture medium was collected, and the fusion protein was purified.

Each fusion protein was purified by performing chromatography using a Mabselect Xtra resin. Equilibration was performed using a protein A binding buffer produced by Thermo Fisher Scientific Inc. Then, the supernatant filtered through a 0.22 um filter was loaded onto the column, and the column was washed using a protein A binding buffer in a volume corresponding to 10 times the volume of the resin. Then, elution was performed using an IgG elution buffer produced by Thermo Fisher Scientific Inc. The fusion protein was collected in a collection tube comprising 20% 1M Tris-HCl at a pH of 9. Then, the buffer for collected fusion protein was replaced by PBS through dialysis.

Then, size exclusion chromatography was performed using a TSKgel G3000SWXL column (TOSOH Bioscience), and absorbance was measured at a wavelength of 214 nm to obtain a high-concentration fusion protein. At this time, the formation of the separated and purified fusion protein was identified by performing SDS-PAGE under reducing (R) or nonreducing (NR) conditions, followed by staining with Coomassie blue.

The amino acid sequence of the produced fusion protein and the nucleic acid sequence encoding the same are shown in Tables 3 and 4 below.

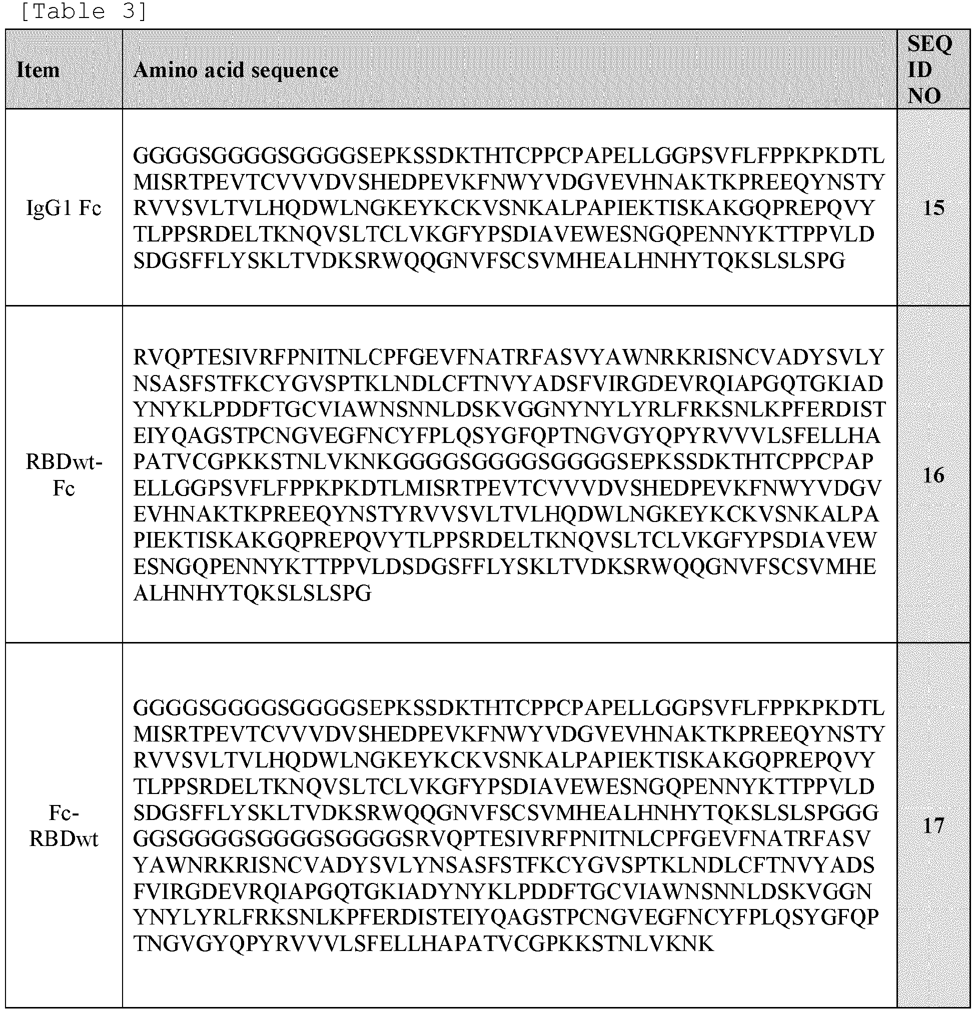

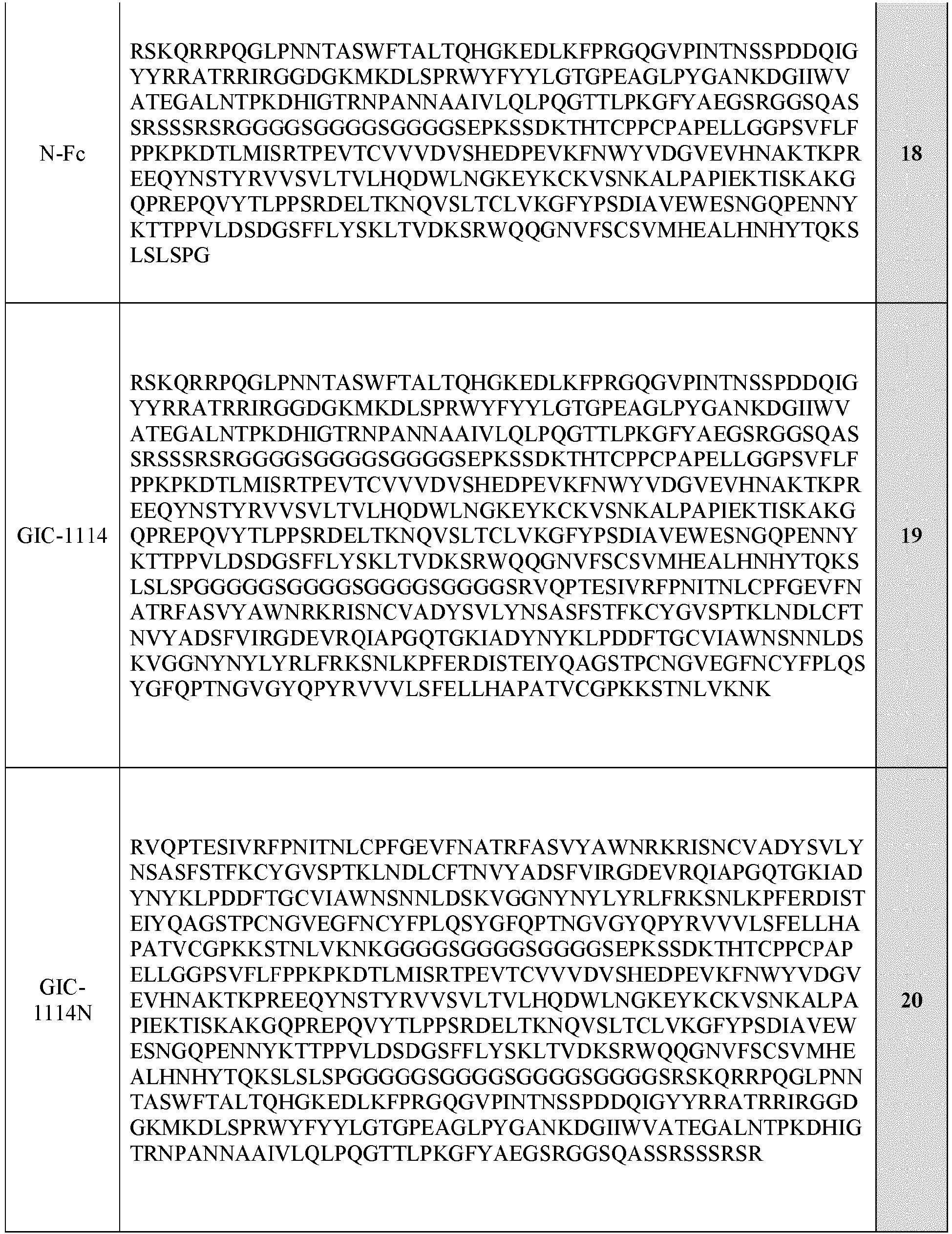

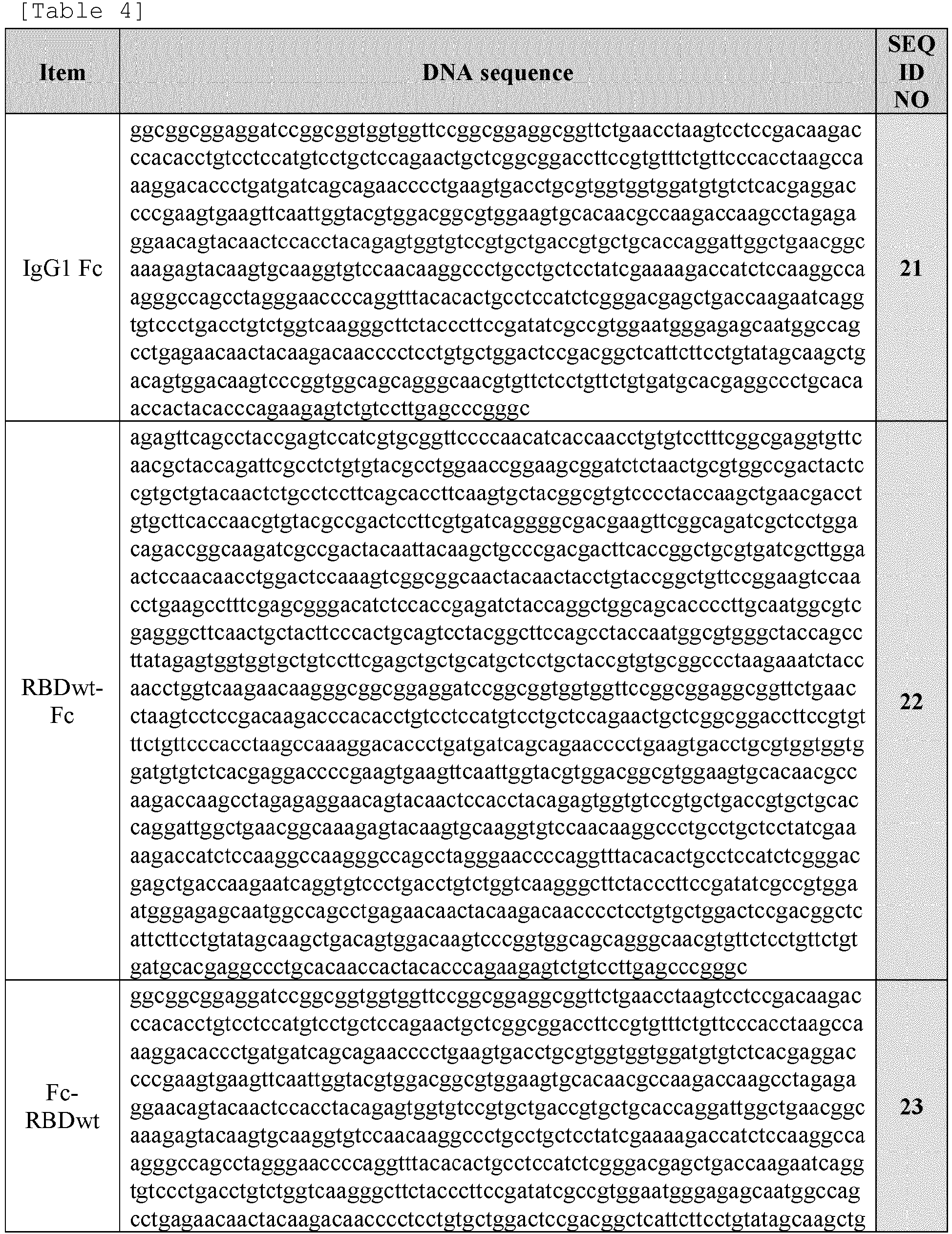

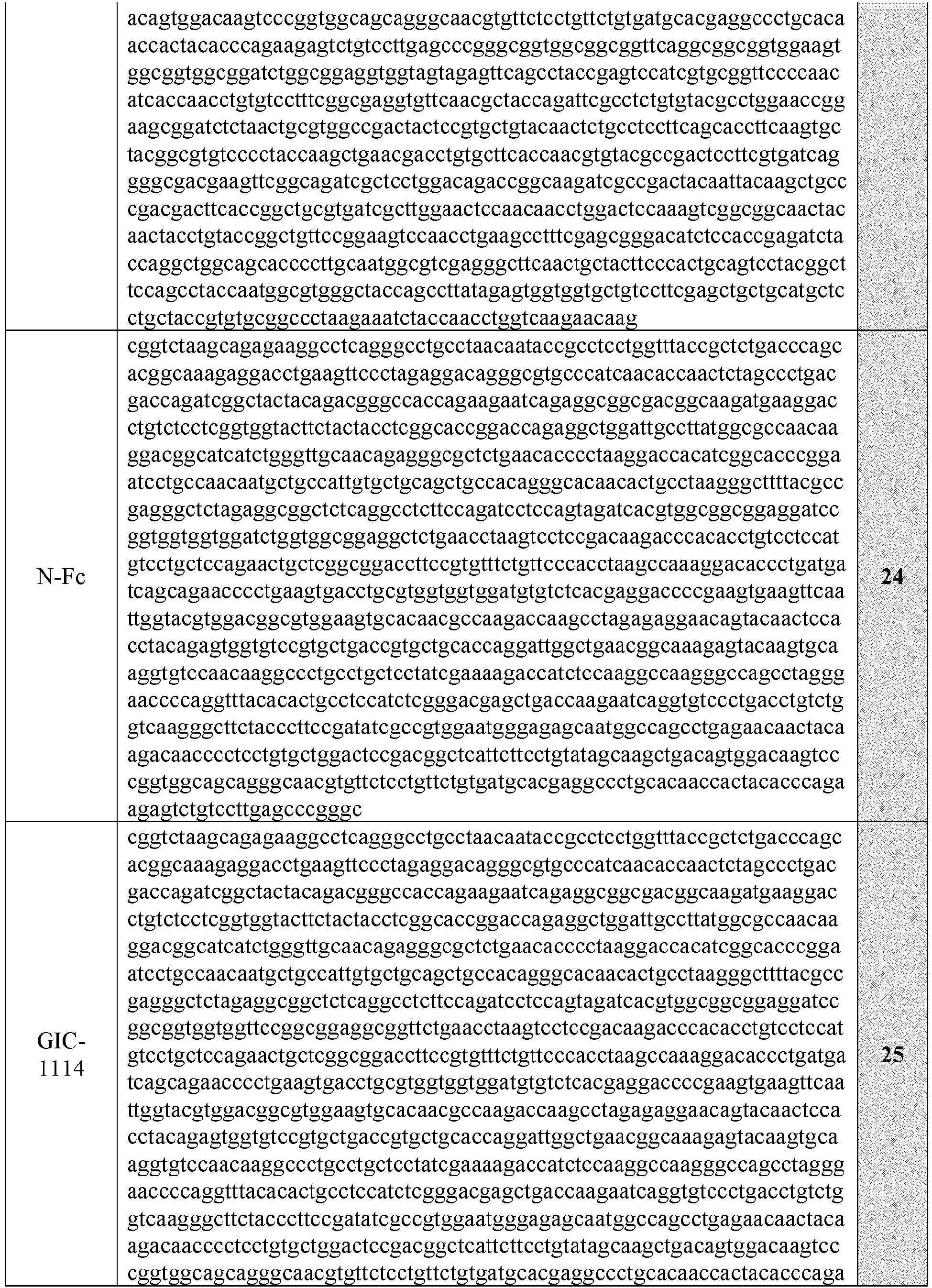

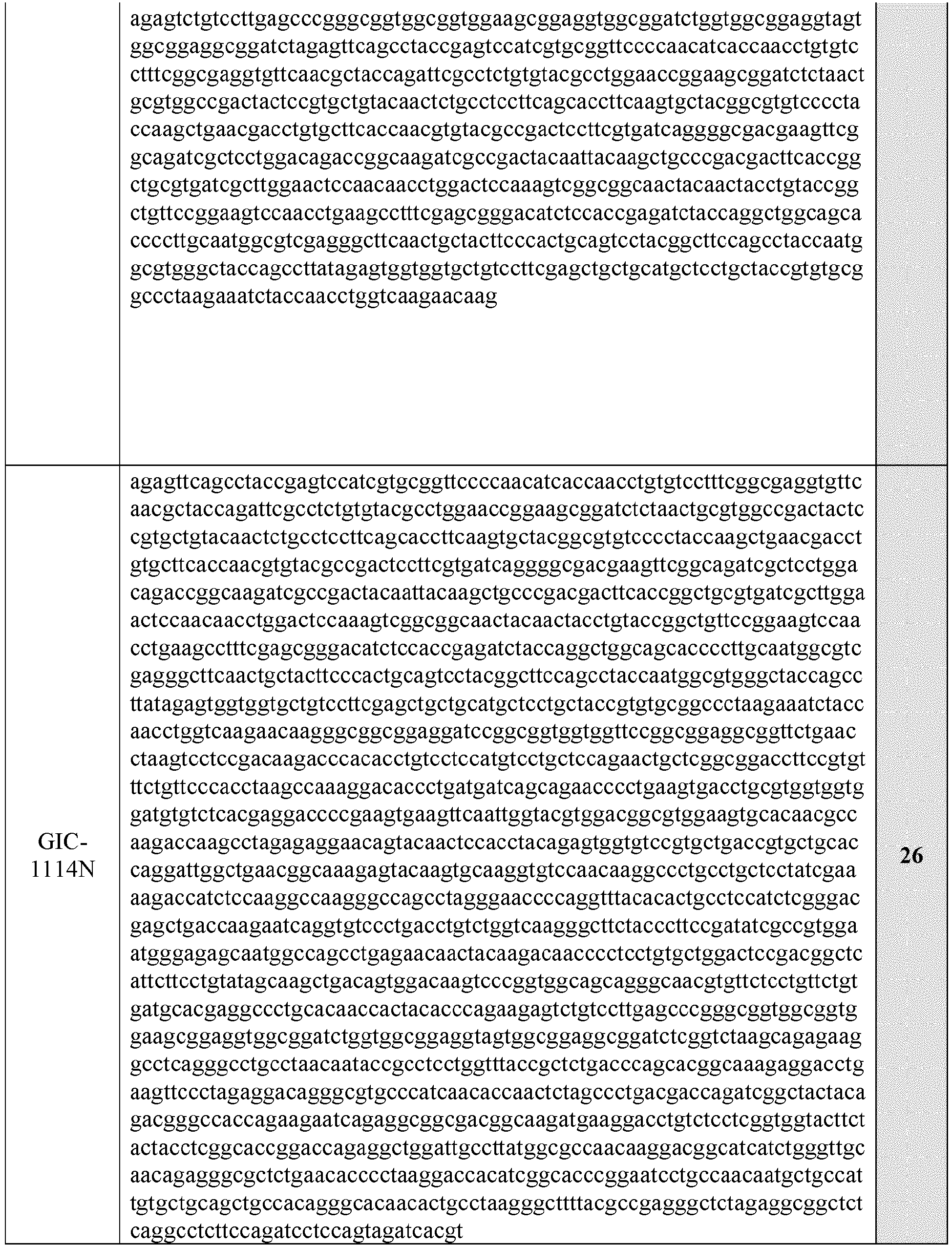

### Example 2: Assessment of immune response upon vaccination of mice with fusion protein of present invention

### Example 4-1: Preparation and vaccination of mice

The mice and the fusion protein used in animal experiments for evaluating the immunogenicity of the fusion protein produced in Example 1 are shown in Table 5 below.

**[Table 5]**

| Animal information | | Strain of origin | Manufacturer | month | Sex |
|---|---|---|---|---|---|
| mouse | | C57BL/6 | ORIENT BIO INC. | 6 weeks | Female |
| Subject information | | Manufacturer | Cat # | Administered Volume | Administered amount |
| subject | IgG1Fc | GI-Cell | N/A | 50 µl | 7.7 µg |
| | GIC-1114 | GI-Cell | N/A | 50 µl | 20 µg |
| | Fc-RBDwt | GI-Cell | N/A | 50 µl | 15 µg |
| | N-Fc | GI-Cell | N/A | 50 µl | 12.5 µg |
| Adjuvant | Addavax | Invivogen | vac-adx-10 | 50 ul | - |
| | MPLA | Invivogen | vac-mpla | 50 ul | 5 µg |
| | alum | Thermo scientific | 77161 | 50 ul | - |

The dosage was controlled to have the same number of moles in consideration of the molecular weight of each fusion protein (IgG1 Fc: 54 kDa, GIC-1114 (N-Fc-RBDwt): 140 kDa, Fc-RBDwt: 105.5 kDa, N-Fc: 87.7 kDa). The fusion protein was administered by intramuscular injection through the femoral muscle, and two weeks after the primary inoculation, the fusion protein was additionally (secondarily) inoculated. 4 weeks after the administration, blood was collected through the abdominal vein of each subject, and the serum was separated and stored in a cryogenic freezer. In addition, the spleen of each subject was harvested at the 4^{th} week and mixed with RPMI1640 medium + 10% FBS, and the resulting mixture was charged in a 15 mL conical tube to prepare a serum in an ice-cold state.

### Example 4-2: Measurement of neutralizing antibodies

ELISA was performed to analyze the titer of mouse neutralizing antibody formation in the serum collected in Example 4-1. Specifically, SARS-CoV-2 (COVID-19) S protein RBD (ACRO biosystem, SPD-C53H3) or human recombinant IgG1 protein (Sinobiologics, 10702-HNAH) was diluted in 1X PBS (10X PBS (Welgene, ML 008-02) 1:10 dilution) and was charged at 100 µl (500 ng/well) in a 96-well plate (BioLegend, 423501), covered with an adhesive plastic, and incubated at 4°C overnight.

A 1X ELISA assay diluent (5X ELISA Assay diluent (BioLegend, 421203), 1X PBS dilution) was added in an amount of 100 µL to each well of the plate and allowed to stand at room temperature for 2 hours while shaking at 100 rpm. The supernatant was removed from each plate, and each well was washed twice with 200 µL of washing buffer (0.05% of Tween20 (Sigma, P7949) in 1X PBS). 254 µL of 1X ELISA assay diluent was added to 2 µL of serum collected from each mouse, after which primary dilution (1:128) was performed. Then, serial dilution to 1:2 1:4, 1:8, 1:16, 1:32, 1:64 and 1:128 was further performed, and 100 µL of the dilution was added to each well, and then allowed to stand at room temperature for 2 hours while shaking at a speed of 100 rpm. The diluted sample was removed and washed 3 times with 200 µL of washing buffer. 100 µL of a goat anti-mouse IgG (H+L) Ab (Invitrogen, 31430) diluent (1X ELISA Assay diluent, 1:10000) was added to each well and incubated in the dark at room temperature for 90 minutes while shaking at 100 rpm. After the supernatant was removed, the residue was washed 5 times with 200 µL of washing buffer. 100 µL of TMB solution (BioLegend, 421101) was added to each well, the reaction was allowed to proceed at room temperature for 1 minute, and 100 µL of a stop solution (BioLegend, 77316) was added to each well to stop the reaction. The OD value of each sample was measured at 450 nm using an ELISA reader (Molecular device, SpectraMax iD3) and the titer of the neutralizing antibody formation was measured.

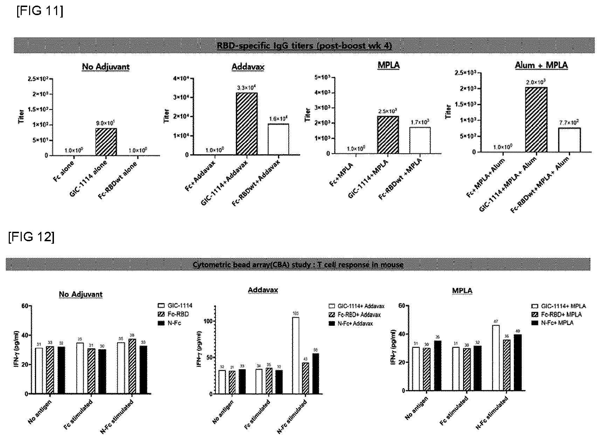

As can be seen from FIG. 11, When vaccination is performed using a fusion protein without adjuvant, a high-antibody RBD-specific IgG titer was obtained only when a fusion protein (GIC-1114) comprising wild-type RBD, nucleocapsid and Fc protein was administered. When vaccination is performed using the IgG1 Fc alone or the Fc-RBDwt fusion protein without an adjuvant, a significant level of neutralizing antibody was not formed.

In addition, when the adjuvant was administered in combination to boost the immune response, the titer of each neutralizing-antibody formation was improved. When the adjuvant was administered in combination, neutralizing antibodies against RBD were formed from the Fc-RBDwt fusion protein, whereas when treated with the GIC-1114 fusion protein, the titer of the neutralizing antibody formation was remarkably high.

The results described above indicate that the fusion protein (GIC-1114) comprising the RBD, N protein and Fc domain of the present invention can exhibit remarkably higher immunogenicity than a fusion protein comprising only the RBD and Fc domain.

### Example 4-3: Assessment of T cell immune response

A cell strainer was placed in a 50 mL conical tube, and 5 mL of 1X PBS (Welgene, ML 008-02) was poured therein to sufficiently wet the cell strainer. A spleen obtained from a mouse was placed in the cell strainer and crushed using a rubber packing part of a 5 mL syringe piston until individual lumps of spleen tissue were no longer visible, and then 20 mL of 1X PBS was slowly poured into the cell strainer to harvest all cells remaining in the cell strainer. The cells harvested in the 50 mL conical tube were centrifuged at 1,500 rpm at 4°C for 5 minutes, the supernatant was discarded, the tube was gently tapped to release the clumped cells and the cells were reacted with 1mL of an ACK lysing buffer (Lonza, Cat# 10-548E) at room temperature for 5 minutes. The reaction product was centrifuged in 4 mL of 1X PBS at 1,500 rpm and 4°C for 5 minutes. The supernatant was discarded and the cells were suspended in 6 mL of R10 media (RPMI 1640 (Welgene, Cat# LM011-01) with 10% FBS (Hyclone, Cat# SY30208.02), 1% antibiotics (Corning, Cat# 30-004-CI) and IL-2 (Novartis, Proleukin) 10 IU), and then the number of cells was measured with an ADAM cell counter (Cat# ADAM-MC2). The cells suspended in R10 media to a density of 1 × 10⁶ cells/well on a 96-well plate and added T cell stimulating antigen(Fc, N-Fc) to 100nM/mL per each wells to adjust the final volume of each well to 200uL. The cells were incubated in an incubator at 37°C and 5% CO₂ for 72 hours, then centrifuged at 1,500 rpm and 4°C for 5 minutes, and the supernatant was harvested and stored at -80°C or lower.

Mouse Th1/Th2/Th17 cytokine capture beads from a mouse Th1/Th2/Th17 cytokine kit (BD, Cat# 560485) were vortexed for 3 to 5 seconds. The number of required samples was calculated, 7 kinds of mouse Th1/Th2/Th17 cytokine capture beads were mixed, and 20 µL thereof was aliquoted into each well. The mixed capture beads were vortexed and loaded to 20 µL into each assay well of a 96-well plate. Standard diluent (mouse Th1/Th2/Th17 cytokine kit (BD, Cat# 560485), 1:2, 1:4, 1:8, 1:16, 1:32, 1:64, 1:128, 1:256) and a negative control solution were each loaded to 20 µL into a 96-well plate, and a sample of the spleen cell supernatant obtained from the mouse stored at -80°C or lower was loaded to 20 µL into each well. 20 µL of Th1/Th2/Th17 PE detection reagent was added and allowed to react in the dark at room temperature for 2 hours. After 2 hours, 200 µL of wash buffer was added into each well, centrifugation was performed at 1,500 rpm and 4°C for 5 minutes, the supernatant was removed, the bead pellet was suspended in 100 µL of washing buffer, and cytokine secretion ability was detected using flow cytometry (FACS).

As can be seen from FIG. 12, the T cells of the mouse spleen vaccinated with GIC-1114 exhibit high IFN-γ levels when stimulated with the N protein (N-Fc) derived from SARS-CoV-2. In particular, when using an adjuvant, the T cells of the mouse spleen vaccinated with GIC-1114 exhibit remarkably improved IFN-γ levels, compared to when treating with other fusion protein (Fc-RBD or N-Fc) having an immune-boosting effect. This means that the fusion protein comprising RBD, Fc and N protein according to the present invention can activate coronavirus-specific cytotoxic T cells.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### Industrial Applicability

The fusion protein comprising the coronavirus-derived receptor-binding domain and the nucleocapsid protein of the present invention is highly applicable to a multivalent vaccine composition having remarkably improved half-life *in vivo,* and remarkably superior efficacy compared to an immunogenic composition comprising only a receptor-binding domain. In particular, the fusion protein of the present invention can greatly improve the titer of the coronavirus-specific antibody formation and T-cell immune response, and is thus useful for the prevention and treatment of coronaviruses comprising SARS-CoV-2.

## Claims

1. A fusion protein comprising:
a coronavirus-derived receptor-binding domain;
a nucleocapsid protein; and
an Fc domain.

2. The fusion protein according to claim 1, wherein the receptor-binding domain comprises a sequence represented by SEQ ID NO: 1.

3. The fusion protein according to claim 1, wherein the nucleocapsid protein comprises a sequence represented by SEQ ID NO: 4.

4. The fusion protein according to claim 1, wherein the Fc domain is an Fc domain of immunoglobulin G (IgG).

5. The fusion protein according to claim 1, wherein the Fc domain comprises a sequence represented by SEQ ID NO: 2.

6. The fusion protein of claim 1, wherein the receptor-binding domain is linked to at least one of an N-terminus and a C-terminus of the Fc domain.

7. The fusion protein according to claim 6, wherein the receptor-binding domain is linked to one of the N-terminus and the C-terminus of the Fc domain, and a nucleocapsid protein is linked to a remaining one thereof.

8. The fusion protein according to claim 1, wherein the fusion protein is a dimer of an Fc domain to which the receptor-binding domain and the nucleocapsid protein are linked.

9. The fusion protein according to claim 1, wherein the coronavirus is SARS-CoV-2.

10. A vaccine composition comprising the fusion protein according to any one of claims 1 to 9.

11. The vaccine composition according to claim 10, further comprising an adjuvant.

12. The vaccine composition according to claim 11, wherein the adjuvant comprises at least one selected from the group consisting of aluminum salts, squalene-containing emulsions, calcium salts, dsRNA analogues, lipopolysaccharides, lipid A analogues, flagellins, imidazoquinolines, CpG ODN, mineral oils, Toll-like receptor (TLR) antagonists, C-type lectin ligands, CD1d ligands, detergents, liposomes, saponins, cytokines and peptides.

13. A pharmaceutical composition for preventing or treating coronavirus infection comprising the fusion protein according to any one of claims 1 to 9 as an active ingredient.

14. The pharmaceutical composition according to claim 13, wherein the coronavirus infection is COVID-19.

15. A nucleic acid encoding the fusion protein according to any one of claims 1 to 9.

16. A recombinant vector comprising the nucleic acid according to claim 15.

17. A host cell into which a nucleic acid encoding the fusion protein according to any one of claims 1 to 9; or a recombinant vector comprising the nucleic acid is introduced.

18. A method for producing a fusion protein comprising:
culturing the host cell according to claim 17 to produce a fusion protein; and
obtaining the produced fusion protein.

19. A method for vaccination against coronavirus infection, the method comprising administering the vaccine composition according to claim 10 to a subject.

20. A method for preventing or treating coronavirus infection, the method comprising administering the pharmaceutical composition according to claim 13 to a subject.

21. Use of the fusion protein according to any one of claims 1 to 9 for manufacture of a vaccine composition.

22. Use of the fusion protein according to any one of claims 1 to 9 for manufacture of a pharmaceutical composition for preventing or treating coronavirus infection.
